# FASCICULE DE BREVET EUROPEEN

(11) **EP 4 186 421 B1**
(45) Date de publication et mention de la délivrance du brevet: **21.05.2025**
(21) Numéro de dépôt: 22210637.9
(22) Date de dépôt: 30.11.2022
(51) Int. Cl.: A61B 5/145

(54) **PROCÉDÉ DE FABRICATION D'UN MODULE ÉLÉMENTAIRE D'UN CAPTEUR À MICROAIGUILLES**
VERFAHREN ZUR HERSTELLUNG EINES ELEMENTAREN MODULS EINES MIKRONADELSENSORS
METHOD FOR PRODUCING AN ELEMENTARY MODULE OF A MICRONEEDLE SENSOR

(30) Priorité: 30.11.2021 FR 2112709
(43) Date de publication de la demande: 31.05.2023
(73) Titulaire: WIZP AS, 3183 Horten (NO)
(72) Inventeur: PIERART, Luc, 94800 VILLEJUIF (FR); HUANG, YULIN, TAIPEI (TW)
(74) Mandataire: Regimbeau

(56) Documents cités:
- WO-A1-2020/023804
- JP-A- 2014 097 163
- US-A1- 2017 319 839
- US-A1- 2018 177 439
- US-A1- 2019 076 075
- US-A1- 2019 201 674
- US-A1- 2021 204 878

## Description

### DOMAINE TECHNIQUE

L'invention concerne les dispositifs prêt-à-porter ou « wearables » utilisés dans des systèmes de surveillance corporelle, par exemple pour le relevé et le suivi de paramètres biochimiques du corps humain, de tels dispositifs comprenant un capteur à micro-aiguilles.

L'invention concerne plus particulièrement la fabrication d'un module élémentaire comprenant au moins deux microaiguilles d'un tel capteur.

### ETAT DE LA TECHNIQUE

La surveillance de nombreuses maladies chroniques connues chez l'humain nécessite un relevé quotidien de paramètres biochimiques. Un niveau de concentration d'un analyte corporel dans un fluide corporel de l'organisme, par exemple dans le plasma sanguin ou dans le liquide interstitiel des cellules de l'organisme, peut être relevé.

A titre d'exemple répandu, le suivi du diabète chez un patient nécessite un relevé précis quotidien de la glycémie du patient.

Une solution pour réaliser le suivi du diabète consiste à réaliser une ponction, par exemple au bout du doigt, pour faire perler une goutte de sang, puis à réaliser une mesure quotidienne de glycémie dans la goutte de sang ainsi obtenue.

Des systèmes de suivi ont été proposés pour se passer de la nécessité d'une ponction manuelle, de sorte à rendre la mesure de glycémie moins laborieuse et moins invasive. On parle de systèmes CGM, pour « Continuous Glucose Monitoring » en anglais.

Certains de ces systèmes CGM réalisent, à intervalles réguliers, une mesure de glycémie au niveau du liquide interstitiel entre les cellules de la peau. La glycémie du liquide interstitiel est très proche de la glycémie du plasma sanguin. Les mesures au niveau du liquide interstitiel permettent un suivi simple et peu invasif de la glycémie des patients ; ces mesures peuvent être réalisées à l'aide de capteurs à aiguille, en transcutané, ou de manière non invasive, comme par exemple en iontophorèse ou en implantable avec une mesure par chimio-fluorescence.

La demande internationale publiée sous le numéro WO 2019/141743 décrit un système de surveillance corporelle, utilisable notamment pour le suivi de la glycémie. Ce système de surveillance comprend une montre électronique attachable au poignet à l'aide d'un bracelet. La montre comporte un boîtier, dans lequel s'insère une capsule amovible interchangeable contenant un capteur à micro-aiguilles et un patch adhésif permettant de maintenir le capteur contre le poignet. Le boitier comprend une batterie qui permet d'alimenter le capteur et le capteur est commandé de manière automatique par l'électronique du boîtier, pour réaliser une mesure transcutanée. La mesure de glycémie par le capteur est une mesure électrochimique.

La fabrication d'un tel capteur nécessite d'avoir un réseau de micro-aiguilles. Cependant il s'avère que l'obtention de réseaux de micro-aiguilles est fastidieuse et conduit souvent à un taux de défaut important en fabrication de ces réseaux. Ceci entraîne une augmentation des coûts de production. Des procédés de fabrication alternatifs sont décrit dans les documents US 2017/319839 A1 et WO 2020/023804 A1.

### EXPOSE DE L'INVENTION

L'invention propose de simplifier la fabrication des réseaux de micro-aiguilles.

A ce titre, l'invention propose un procédé de fabrication d'un module élémentaire d'un capteur à micro-aiguilles, le procédé étant tel que défini dans la revendication indépendante 1, et comprenant, inter alia, une étape d'assemblage d'un support avec au moins une agrafe, l'assemblage consistant à introduire l'agrafe dans une ouverture formée dans toute l'épaisseur du support, l'agrafe formant des micro-aiguilles.

L'invention est avantageusement complétée par les caractéristiques suivantes, prises seules ou en une quelconque de leur combinaison techniquement possible :
- les branches de l'agrafe sont en forme de pointe ou biseautée de manière à faciliter l'insertion des agrafes dans la peau d'un utilisateur ;
- l'agrafe est fixée au support par introduction en force dans l'ouverture ou par collage ou par clipsage ;
- l'agrafe est obtenue par la mise en œuvre des étapes suivantes : découpe dans une plaque d'un ruban d'agrafes, chaque agrafe étant attachée à une bande par un pied de découpage ;
- dépôt d'au moins une couche conductrice sur le ruban d'agrafes, des pieds et de la bande ;
- séparation de chaque agrafe au niveau du pied de découpage ;
- le pied de découpage s'étend depuis une zone centrale du bras de chaque agrafe vers la bande, le pied étant attaché au bras sur une zone de liaison de taille inférieure à la longueur du bras ;
- la couche conductrice est de l'or ou du palladium ou du titane ;
- un substrat comprenant un réseau de supports de modules élémentaires, le procédé comprenant après l'assemblage des modules élémentaires, un découpage de chaque module élémentaire ;
- l'agrafe présente une épaisseur comprise entre 100 microns et 600 microns, de préférence 250 microns, une largeur de bras comprise entre 1500 et 3000 microns, de préférence, 2000 microns, une hauteur de bras comprise entre 200 microns et 1500 microns, de préférence 800 microns.

L'invention propose aussi un module élémentaire comprenant un support et au moins une agrafe, le module élémentaire étant obtenu au moyen d'un procédé selon l'invention.

L'invention propose aussi un capteur à microaiguilles comprenant une platine et au moins un module élémentaire obtenu au moyen d'un procédé selon l'invention.

L'invention permet d'obtenir simplement des réseaux de microaiguilles.

Avec un tel procédé il n'y pas de limitation pour la hauteur des agrafes, les autres techniques de fabrication de micro-aiguilles ne permettant pas simplement d'obtenir des hauteurs supérieures à 500 microns.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre une vue d'ensemble d'un dispositif de surveillance d'un analyte corporel

la figure 2 illustre une vue schématique d'un capteur à aiguilles selon l'invention ;
la figure 3a et la figure 3b illustrent un module élémentaire selon deux modes de réalisation de l'invention ;
la figure 4a et la figure 4b illustrent un support d'un module élémentaire selon deux modes de réalisation de l'invention ;
la figure 5a et la figure 5b illustrent respectivement une agrafe d'un module élémentaire selon un mode de réalisation de l'invention et deux agrafes en croix selon un autre mode de réalisation de l'invention ;
la figure 6a, la figure 6b et la figure 6c illustrent différentes variantes d'une agrafe d'un module élémentaire selon un mode de réalisation de l'invention ;
la figure 7 illustre des étapes d'un procédé de fabrication d'un module élémentaire selon un mode de réalisation de l'invention :
la figure 8 un substrat comprenant plusieurs supports de module élémentaire selon un mode de réalisation de l'invention ;
la figure 9 illustre des étapes de fabrication d'une agrafe d'un module élémentaire selon un mode de réalisation de l'invention.

Sur l'ensemble des figures les éléments similaires portent des références identiques.

### DESCRIPTION DETAILLEE

### Architecture générale d'un dispositif de surveillance corporelle

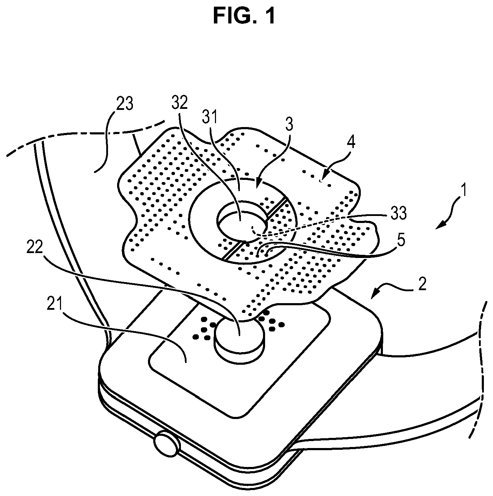

La **figure 1** illustre un dispositif 1 de surveillance corporelle comprenant un boitier 2, un capteur 3 et un patch adhésif 4.

Le capteur 3 est ici un capteur à aiguilles prévu pour fournir une mesure de courant électrique au sein du liquide interstitiel du porteur du dispositif 1.

Des aiguilles 5 sont avantageusement disposées sur une face interne 31 du capteur 3. Cette face interne 31 est destinée à être placée sur la peau du porteur.

Le capteur 3 est assemblé au patch adhésif 4 constituent ensemble une capsule. Le capteur 3 peut aussi être amovible par rapport au patch 4. Une telle capsule est avantageusement montée amovible avec le boitier 2. En particulier, la capsule et donc le capteur 3 s'engage de manière préférée dans une cavité 21 du boitier 2 située sur sa face destinée à être en contact avec la peau. Le capteur 3 comprend une face externe 32 opposée à la face interne 31.

Le boîtier 2 et la capsule peuvent présenter des formes complémentaires, ce qui limite l'effort nécessaire pour la bonne insertion de la capsule contre le boîtier 2.

Le patch 4 comporte une couche adhésive, ou est lui-même formé en un matériau adhésif. Le patch permet donc l'attache de la capsule à la peau du porteur, et favorise le maintien des aiguilles 5 dans le liquide interstitiel. Le patch 4 présente par exemple une forme annulaire, et recouvre la capsule.

Le capteur 3 ici illustré est de forme circulaire avec un orifice central 33 mais elle peut prendre d'autres formes : rectangulaire, oblongue, ellipsoïdale avec ou sans orifice central. L'orifice central 33 permet de positionner correctement le capteur 3 dans la cavité 21 du boitier qui comprend un plot central de positionnement (non représenté).

Le capteur 3 comprend donc des éléments qui permettent de prélever le liquide soit d'amener les signaux détectés par chaque microaiguille vers le boitier 2 pour traitement (non décrit ici).

Le capteur 3 peut prendre la forme d'une plaque de plastique, d'un circuit imprimé (rigide ou flexible en silicium), d'une plaque en métal non conducteur comme par exemple de l'aluminium.

Le patch adhésif 4 est adapté pour être collé à la peau et supporte le capteur 3 et permet de détacher le boitier 2 sans enlever le capteur 3 en le gardant collée au corps. Une telle configuration permet d'éviter d'enlever le capteur pour certaines opérations qui n'implique que le boitier : rechargement de la batterie, réparation, remplacement, extraction des données vers un ordinateur.

Le boitier 2 est avantageusement en forme de boitier de montre et comprend des moyens d'attaches 23 du dispositif au poignet d'un utilisateur. Il s'agit notamment d'un bracelet adapté pour entourer le poignet d'un utilisateur. Le bracelet est de préférence réglable.

Le boitier 2 loge plusieurs éléments permettant d'analyser ou d'extraire du liquide interstitiel. A ce titre, on pourra se référer au document WO 2019/141743 au nom du déposant qui décrit en détail la mesure et la détection d'une grandeur physique à partir de microaiguilles au contact d'un liquide corporel pouvant être prélevé ou non.

Avantageusement, la montre comprend en outre une interface de communication sans fil, par exemple via un réseau de télécommunications de type 3G et/ou 4G et/ou 5G et/ou Wi-Fi et/ou Bluetooth et/ou NFC et/ou DECT.

Également, la montre peut comprendre un indicateur lumineux tel qu'une diode, qui peut être utilisé pour signaler la fin d'une opération de préparation du capteur.

Les aiguilles 5 sont avantageusement des micro-aiguilles. Le capteur 3 comporte de préférence entre quatre et cinquante micro-aiguilles voire quatre cent microaiguilles. Bien entendu, un nombre différent peut être considéré sans que cela ne limite la description de l'invention ici faite.

On entend par micro-aiguille, une aiguille présentant une hauteur faible de préférence entre 10 µm et 1000 µm, de préférence entre 0,3 mm et 0,8mm. La hauteur des micro-aiguilles est suffisamment faible pour éviter le contact avec un nerf de douleur mécanique du porteur lorsque le dispositif est porté.

Les microaiguilles 5 permettent de mesurer le liquide corporel.

Les microaiguilles 5 sont pleine pour analyser directement le liquide. Pour analyser du liquide, les microaiguilles ne prélèvent pas de liquide et intègrent le capteur sur leur surface sous la forme d'un revêtement tel qu'un matériau biochimique apte à réagir avec l'analyse à effectuer sur le liquide.

La longueur des aiguilles 5 est ainsi suffisamment réduite pour éviter le contact avec un nerf de l'utilisateur, pour limiter la douleur causée par le port du dispositif 1.

Chaque aiguille présente par exemple une forme pyramidale.

Dans le présent exemple, chaque aiguille 5 comprend à sa surface au moins un matériau chimique ou biochimique apte à réagir avec l'analyte corporel dont on souhaite obtenir une mesure (c'est-à-dire ici le glucose). Un matériau apte à réagir avec l'analyte corporel est par exemple une enzyme capable d'oxyder l'analyte corporel.

De manière avantageuse, le capteur 3 comprend plusieurs microaiguilles qui consistent en un réseau de microaiguilles en ce qu'elles sont électriquement connectées entre elles par groupe. Les microaiguilles transpercent la peau pour venir au contact du liquide interstitiel lorsque le capteur est au contact de la peau.

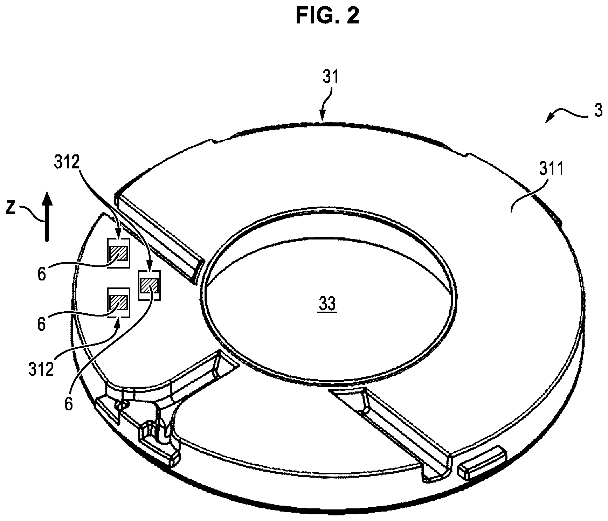

Le capteur 3 illustré sur la figure 2 comprend, un substrat 311 doté de plusieurs modules élémentaires 6 chaque module élémentaire comprenant un support 61 et au moins deux microaiguilles (non visibles). Les modules élémentaires sont reliés entre eux au moyen de pistes métalliques. De manière avantageuse, chaque module élémentaire 6 est positionné dans un logement 312 et est relié au substrat 311 par exemple par collage.

Ainsi le capteur 3 comprend des électrodes, chacune constituée par au moins une microaiguille. En particulier, le capteur 3 comporte une électrode de conductivité de travail et une électrode de conductivité de référence 314.

En cours d'utilisation du capteur 3 pour réaliser une mesure, une tension est générée entre plusieurs aiguilles. Au moins une partie des aiguilles 5 du capteur 3 sont au moins partiellement immergées dans le liquide interstitiel. Le matériau chimique ou biochimique présent à la surface des aiguilles 5 réagit avec le glucose du liquide interstitiel.

Le capteur 3 fournit ainsi une mesure de courant électrique, représentative de la concentration en glucose dans le liquide interstitiel.

Le substrat 311 et les aiguilles 5 sont préférentiellement disposés sur une unique face du capteur 3, qui est la face dirigée vers le haut selon l'orientation de la figure 2. Cette face haute est destinée à être disposée face à la peau de l'utilisateur.

Chaque aiguille s'étend de la face haute selon une direction Z, depuis sa base jusqu'à sa pointe. La direction Z est de préférence orthogonale à un plan de la face haute.

L'ouverture 33 centrale est ici de forme circulaire. Le capteur 3 présente ainsi, dans le présent exemple, une forme générale annulaire.

### Module élémentaire

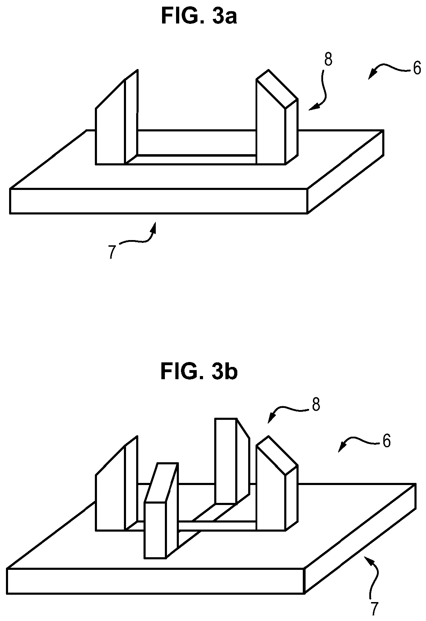

La **figure 3a** illustre un module 6 élémentaire comprenant un support 7 et une agrafe 8 positionnée dans une ouverture formée dans toute l'épaisseur du support 7. L'agrafe 8 forme deux microaiguilles pour le capteur 3 ci-dessus décrit.

La **figure 3b** illustre un module 6 élémentaire comprenant un support 7 et deux agrafes 8 positionnées en croix dans une ouverture formée dans toue l'épaisseur du support 7.

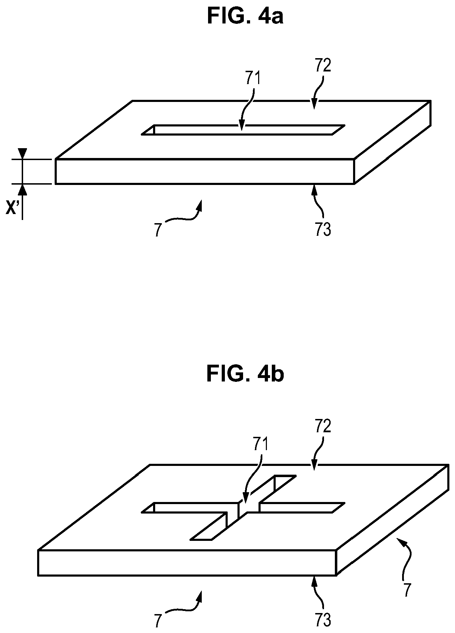

Comme illustré sur les **figures 4a** et **4b****,** le support 7 et l'ouverture sont par exemple de forme parallélépipédique mais d'autres formes sont envisageables.

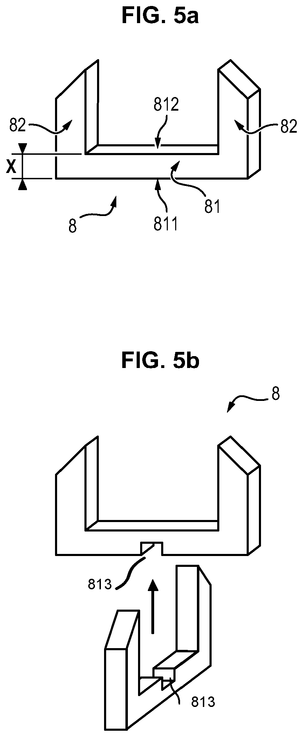

La **figure 5a** illustre une agrafe 8 selon un mode de réalisation. L'agrafe 8 est de préférence en forme de U et comprend un bras 81 et deux branches 82 s'étendant depuis le bras 81. De préférence, les branches 82 s'étendent depuis les extrémités du bras 81. Sur la **figure 5b** lorsqu'il s'agit de positionner en croix deux agrafes on prévoit des marches 813 sur les bras 81, ces marches sont complémentaires.

Le bras 81 comprend une épaisseur X inférieure ou égale à l'épaisseur X' du substrat.

En outre, l'agrafe 8 est positionnée dans l'ouverture 71 de sorte à aligner le plus possible la surface inférieure 811 du bras avec la surface inférieure 73 du support 7 mais aussi à veiller que le bras 81 ne dépasse pas de la surface supérieure 72 du support 7 celle qui doit être en contact avec la peau de l'utilisateur. En effet, le module 6 élémentaire est destiné à être en contact avec la peau de l'utilisateur et le bras 81 ne doit pas « sortir » de l'ouverture 71 ce qui pourrait blesser l'utilisateur. Par ailleurs, au niveau de la surface inférieure 73 du support 7, il faut pouvoir bien fixer le module 6 élémentaire au substrat 311 du capteur 3, les surfaces en contact devant être les plus planes possibles pour optimiser la fixation du module 6 élémentaire.

L'agrafe 8 en forme de U présente des branches 82 parallèles ou quasi parallèle. La notion d'agrafe est bien connue de l'homme du métier de sorte que la structure en tant que telle de l'agrafe lui est bien connue.

En particulier, la section du bras 81 et des branches est rectangulaire comme visible sur la figure 5a qui illustre une agrafe.

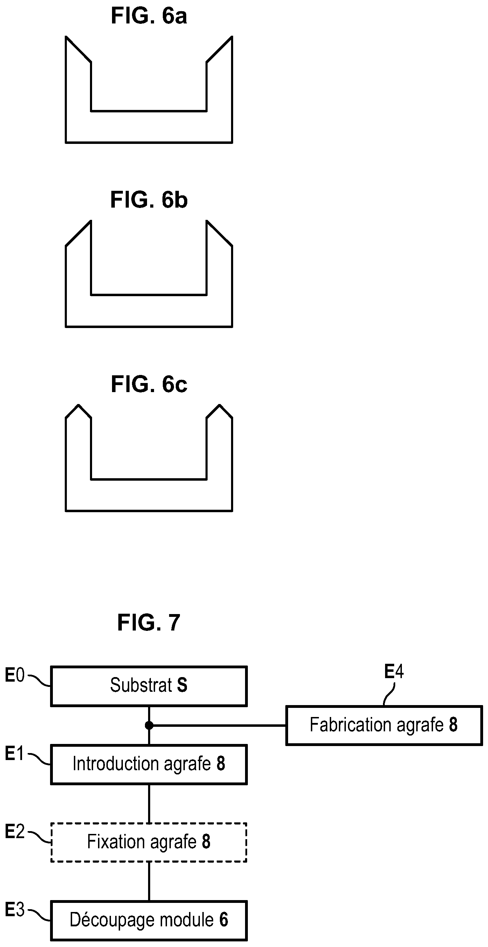

Comme illustré sur les **figures 6a, 6b** et **6c** les branches 82 de l'agrafe 8 sont en forme de pointe ou biseautée de manière à faciliter l'insertion des agrafes dans la peau d'un utilisateur. Dans le cas des branches biseautées, c'est l'extrémité libre des branches 82 qui est taillée en biseau. Ce biseau peut être orienté soit vers l'extérieur soit vers l'intérieur du bras 81.

La forme de l'extrémité de l'agrafe 8 est adaptée à l'insertion dans la peau de l'utilisateur puisqu'elle a la fonction de micro-aiguille.

L'agrafe 8 présente des dimensions adaptées à sa fonction de microaiguilles et présente une épaisseur comprise entre 100 microns et 600 microns, de préférence 250 microns, une largeur de bras comprise entre 1500 et 3000 microns, de préférence, 2000 microns, une hauteur de bras comprise entre 200 microns et 1500 microns, de préférence 800 microns.

L'agrafe 8 est de base métallique et comporte un dépôt conducteur d'or, de platine ou de titane. En effet, s'agissant de microaiguilles pour un capteur biochimique, il faut prévoir une couche permettant à un enzyme d'être fixé à l'agrafe.

L'utilisation d'une agrafe 8 est avantageuse en ce que la connexion électrique entre les branches 82 et le bras 81 est assurée de manière continue. Les branches 82 sont en effet destinées à être en contact avec le liquide comprenant l'analyte à mesurer, un courant étant alors généré au niveau de l'agrafe 8, courant qui doit être amené au boitier du capteur 3 pour traitement. L'utilisation de l'agrafe 8 évite de prévoir des connexions électriques complexes.

### Fabrication d'un module élémentaire

La **figure 7** illustre des étapes d'un procédé de fabrication d'un module élémentaire conforme à un mode de réalisation de l'invention.

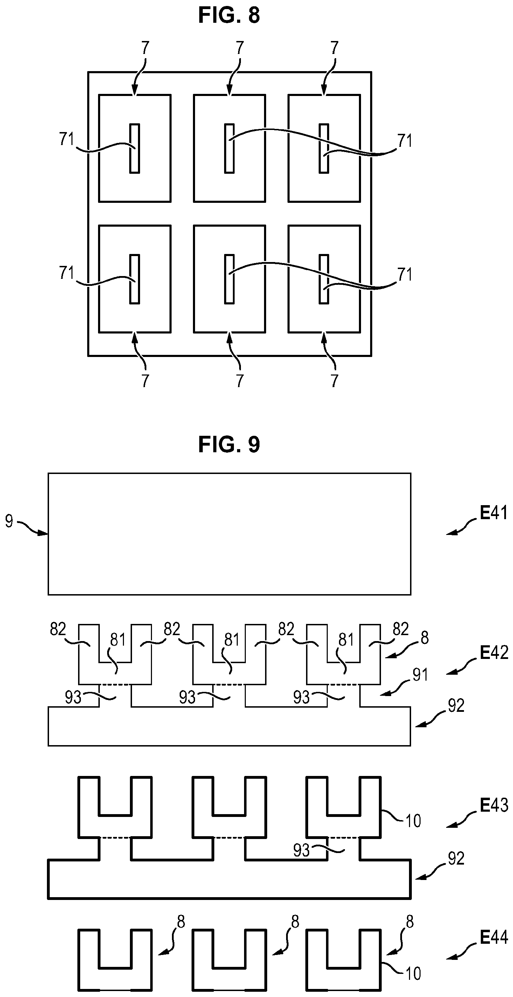

En relation avec la **figure 8****,** pour fabriquer un module élémentaire 6, on part d'un substrat S comprenant un réseau de supports 7 de modules 6 élémentaires (étape E0). Chaque support 7 comprend une ouverture 71 de forme rectangulaire (ou plus ou moins rectangulaire). L'ouverture 71 est adaptée à recevoir une agrafe 8 comme précédemment décrite.

Bien entendu, on peut aussi partir d'un seul support 7 pour n'obtenir qu'un module élémentaire 6.

Dans chaque ouverture, une agrafe 8 est introduite (étape E1) L'introduction de l'agrafe 8 peut se faire en force de sorte que la seule introduction permet de fixer l'agrafe au support. Alternativement après l'introduction, l'agrafe est fixée au support par collage ou pas clipsage (étape E2). Les agrafes sont obtenues au préalable de préférence via une fabrication spécifique (étape E4) (voir ci-après).

Une fois que les agrafes sont introduites dans les supports, un découpage de chaque module 6 élémentaire du substrat est effectué (étape E3)

On obtient alors des modules élémentaires comme décrit ci-avant.

Ces différentes étapes s'appliquent à un module élémentaire comprenant deux agrafes comme présentées ci-dessus.

### Fabrication des agrafes

Les agrafes sont obtenues de manière simple et peu complexe.

En effet, comme illustré sur la **figure 9** on part d'une plaque 9 (en métal) (étape E41). Dans cette plaque 9 (en métal) un ruban d'agrafes est découpé (étape 42). Chaque agrafe 8 est attachée à une bande 92 par un pied de découpage 93, le pied de découpage s'étendant depuis le bras de l'agrafe dans une direction opposée à celle des branches. Le pied 93 de découpage s'étend depuis une zone centrale du bras 81 de chaque agrafe 8 vers la bande 92, le pied 93 étant attaché au bras 81 sur une zone de liaison de taille inférieure à la longueur du bras 82.

Ensuite, un dépôt d'au moins une couche conductrice 10 sur le ruban d'agrafes, des pieds et de la bande est effectuée (étape E43). Ici il s'agit de déposer un conducteur tel que de l'or, du platine ou du titane.

Ensuite, chaque agrafe 9 est séparée au niveau du pied de découpage (étape E44).

L'intérêt de prévoir un pied du découpage est que l'étape de dépôt s'effectue de manière large sur tout le ruban et qu'au moment de la séparation de l'agrafe 8, seule la partie de l'agrafe 8 connectée au pied de découpage 93 ne comporte plus le dépôt additionnel conducteur 10. Ainsi, la connexion électrique depuis les branches vers le bras est assurée.

Sur la figue 9 on a représenté le dépôt conducteur par un trait plus large que celui de la zone centrale du bras 81. On voit la partie en trait fin qui correspond à la partie sans dépôt additionnel et qui correspond à la zone de découpage permettant de séparer chaque agrafe 8.

## Revendications

1. Procédé de fabrication d'un module élémentaire (6) d'un capteur à micro-aiguilles, comprenant une étape d'assemblage d'un support (7) avec au moins une agrafe (8), le support comprenant une surface supérieure (72) destinée à être en contact avec la peau d'un utilisateur et une surface inférieure (73), l'agrafe est en forme de U et comprend un bras (81) et deux branches (82) s'étendant depuis chaque extrémité du bras (81), le bras comprenant une épaisseur (X) inférieure ou égale à l'épaisseur (X') du support (7), les extrémités des branches étant adaptées à être insérées dans la peau d'un utilisateur et forment deux micro-aiguilles, l'assemblage consistant à introduire l'agrafe (8) dans une ouverture (71) rectangulaire formée dans toute l'épaisseur du support (7) de manière à positionner l'agrafe de sorte que le bras (81) ne dépasse pas de la surface supérieure (72) du support (7) et à aligner une surface inférieure du bras avec une surface inférieure du support, et à fixer l'agrafe (8) au support, l'agrafe formant des micro-aiguilles, un module élémentaire comprenant au moins deux micro-aiguilles pleines.

2. Procédé selon la revendication 1, dans lequel les branches (82) de l'agrafe (8) sont en forme de pointe ou biseautée de manière à faciliter l'insertion des agrafes dans la peau d'un utilisateur.

3. Procédé selon l'une quelconque des revendications précédentes, dans lequel l'agrafe (8) est fixée au support par introduction en force dans l'ouverture (71) ou par collage ou par clipsage.

4. Procédé selon l'une quelconque des revendications précédentes, dans lequel l'agrafe (8) est obtenue par la mise en œuvre des étapes suivantes
- découpe (E42) dans une plaque (9) d'un ruban (91) d'agrafes (8), chaque agrafe (8) étant attachée à une bande (91) par un pied (93) de découpage ;
- dépôt (E43) d'au moins une couche (10) conductrice sur le ruban d'agrafes, des pieds et de la bande ;
- séparation (E44) de chaque agrafe (8) au niveau du pied de découpage.

5. Procédé selon la revendication précédente, dans lequel le pied (93) de découpage s'étend depuis une zone centrale du bras (81) de chaque agrafe (8) vers la bande (92), le pied (93) étant attaché au bras (81) sur une zone de liaison de taille inférieure à la longueur du bras (82).

6. Procédé selon l'une quelconque des revendications 4 à 5, dans lequel la couche (10) conductrice est de l'or ou du palladium ou du titane.

7. Procédé selon l'une quelconque des revendications précédentes, dans lequel l'assemblage est mis en œuvre sur un substrat (11) comprenant un réseau de supports de modules élémentaires, le procédé comprenant après l'assemblage des modules élémentaires, un découpage de chaque module élémentaire.

8. Procédé selon l'une quelconque des revendications précédentes, dans lequel l'agrafe (8) présente une épaisseur comprise entre 100 microns et 600 microns, de préférence 250 microns, une largeur de bras comprise entre 1500 et 3000 microns, de préférence, 2000 microns, une hauteur de bras comprise entre 200 microns et 1500 microns, de préférence 800 microns.

9. Module élémentaire comprenant un support et au moins une agrafe (8), le module élémentaire étant obtenu au moyen d'un procédé selon quelconque des revendications précédentes.

10. Capteur à microaiguilles comprenant une platine et au moins un module élémentaire selon la revendication 9.

## Patentansprüche

1. Verfahren zur Herstellung eines elementaren Moduls (6) eines Mikronadelsensors, umfassend einen Schritt des Zusammenbauens eines Trägers (7) mit mindestens einer Klammer (8), wobei der Träger eine obere Fläche (72), die dazu bestimmt ist, mit der Haut eines Benutzers in Kontakt zu sein, und eine untere Fläche (73) umfasst, die Klammer U-förmig ist und einen Arm (81) und zwei Schenkel (82) umfasst, die sich von jedem Ende des Arms (81) erstrecken, wobei der Arm eine Dicke (X) umfasst, die kleiner oder gleich der Dicke (X') des Trägers (7) ist, wobei die Enden der Schenkel geeignet sind, in die Haut eines Benutzers eingeführt zu werden und zwei Mikronadeln bilden, wobei der Zusammenbau darin besteht, die Klammer (8) in eine rechteckige Öffnung (71) einzuführen, die in der gesamten Dicke des Trägers (7) derart ausgebildet ist, dass die Klammer derart positioniert wird, dass der Arm (81) nicht über die obere Fläche (72) des Trägers (7) hinausragt und eine untere Fläche des Arms mit einer unteren Fläche des Trägers ausgerichtet ist und die Klammer (8) am Träger befestigt wird, wobei die Klammer Mikronadeln bildet, wobei ein elementares Modul mindestens zwei volle Mikronadeln umfasst.

2. Verfahren nach Anspruch 1, wobei die Schenkel (82) der Klammer (8) spitz oder abgeschrägt sind, um das Einführen der Klammern in die Haut eines Benutzers zu erleichtern.

3. Verfahren nach einem der vorhergehenden Ansprüche, wobei die Klammer (8) durch gewaltsames Einführen in die Öffnung (71) oder durch Kleben oder durch Klemmen am Träger befestigt wird.

4. Verfahren nach einem der vorhergehenden Ansprüche, wobei die Klammer (8) durch die Durchführung der folgenden Schritte erhalten wird
- Schneiden (E42) eines Streifens (91) von Klammern (8) aus einer Platte (9), wobei jede Klammer (8) durch einen Schneidfuß (93) an einem Band (91) befestigt ist;
- Aufbringen (E43) mindestens einer leitenden Schicht (10) auf den Streifen von Klammern, Füßen und des Bandes;
- Abtrennen (E44) jeder Klammer (8) im Bereich des Schneidfußes.

5. Verfahren nach vorhergehendem Anspruch, wobei sich der Schneidfuß (93) von einem mittleren Bereich des Arms (81) jeder Klammer (8) zum Band (92) erstreckt, wobei der Fuß (93) an einem Verbindungsbereich, der kleiner als die Armlänge (82) ist, am Arm (81) befestigt ist.

6. Verfahren nach einem der Ansprüche 4 bis 5, wobei die leitende Schicht (10) aus Gold oder Palladium oder Titan besteht.

7. Verfahren nach einem der vorhergehenden Ansprüche, wobei das Zusammenbauen auf einem Substrat (11) durchgeführt wird, das ein Netzwerk von Trägern für elementare Module umfasst, wobei das Verfahren nach dem Zusammenbauen der elementaren Module ein Schneiden jedes elementaren Moduls umfasst.

8. Verfahren nach einem der vorhergehenden Ansprüche, wobei die Klammer (8) eine Dicke zwischen 100 Mikron und 600 Mikron, vorzugsweise 250 Mikron, eine Armbreite zwischen 1500 und 3000 Mikron, vorzugsweise 2000 Mikron, eine Armhöhe zwischen 200 Mikron und 1500 Mikron, vorzugsweise 800 Mikron, aufweist.

9. Elementares Modul, das einen Träger und mindestens eine Klammer (8) umfasst, wobei das elementare Modul durch ein Verfahren nach einem der vorhergehenden Ansprüche erhalten wird.

10. Mikronadel-Sensor, der eine Platine und mindestens ein elementares Modul nach Anspruch 9 umfasst.

## Claims

1. A method for manufacturing a basic module (6) of a microneedle sensor, comprising a step of assembling a support (7) with at least one staple (8), the support comprising an upper surface (72) intended to be in contact with the skin of a user and a lower surface (73), the staple being U-shaped and comprises an arm (81) and two branches (82) extending from each end of the arm (81), the arm comprising a thickness (X) less than or equal to the thickness (X') of the support (7), the ends of the branches being adapted to be inserted into the skin of a user and forming two microneedles, the assembly consisting of introducing the staple (8) into a rectangular opening (71) formed through the entire thickness of the support (7) so as to position the staple so that the arm (81) does not protrude from the upper surface (72) of the support (7) and to align a lower surface of the arm with a lower surface of the support, and to fix the staple (8) to the support, the staple forming microneedles, a basic module comprising at least two solid microneedles.

2. The method according to claim 1, wherein the branches (82) of the staple (8) are pointed or beveled to facilitate the insertion of the staples into the skin of a user.

3. The method according to any one of the preceding claims, wherein the staple (8) is fixed to the support by force-fitting into the opening (71) or by gluing or clipping.

4. The method according to any one of the preceding claims, wherein the staple (8) is obtained by implementing the following steps:
∘ cutting (E42) in a plate (9) of a strip (91) of staples (8), each staple (8) being attached to a strip (91) by a cutting foot (93);
∘ depositing (E43) at least one conductive layer (10) on the strip of staples, the feet and the strip;
∘ separating (E44) each staple (8) at the cutting foot.

5. The method according to the preceding claim, wherein the cutting foot (93) extends from a central area of the arm (81) of each staple (8) towards the strip (92), the cutting foot (93) being attached to the arm (81) on a connection area smaller than the length of the arm (82).

6. The method according to any one of claims 4 to 5, wherein the conductive layer (10) is gold or palladium or titanium.

7. The method according to any one of the preceding claims, wherein the assembly is implemented on a substrate (11) comprising a network of basic module supports, the method comprising, after assembling the basic modules, cutting each basic module.

8. The method according to any one of the preceding claims, wherein the staple (8) has a thickness between 100 microns and 600 microns, preferably 250 microns, an arm width between 1500 and 3000 microns, preferably 2000 microns, an arm height between 200 microns and 1500 microns, preferably 800 microns.

9. A basic module comprising a support and at least one staple (8), the basic module being obtained by a method according to any one of the preceding claims.

10. A microneedle sensor comprising a plate and at least one basic module according to claim 9.
